# EUROPEAN PATENT SPECIFICATION

(11) **EP 4 489 708 B1**
(45) Date of publication and mention of the grant of the patent: **17.12.2025**
(21) Application number: 23713028.1
(22) Date of filing: 06.03.2023
(51) Int. Cl.: A61F 13/472, A61F 13/537

(54) **METHOD OF FOLDING A FEMININE HYGIENE ARTICLE**
VERFAHREN ZUM FALTEN EINES HYGIENARTIKELS FÜR FRAUEN
MÉTHODE DE PLIAGE D'UN ARTICLE D'HYGIÈNE FÉMININE

(30) Priority: 09.03.2022 US 202263317981 P
(43) Date of publication of application: 15.01.2025
(73) Proprietor: The Procter & Gamble Company, Cincinnati, OH 45202 (US)
(72) Inventor: VOHWINKEL, Henning, 65824 Schwalbach Am Taunus (DE)
(74) Representative: P&G Patent Germany
(86) International application number: PCT/US2023/063771
(87) International publication number: WO 2023/172866

(56) References cited:
- WO-A1-96/12460
- US-A1- 2016 310 330
- US-A1- 2019 314 211
- US-A1- 2020 375 810

## Description

### FIELD OF THE INVENTION

The present application pertains to absorbent articles and more particularly to a method of folding a feminine hygiene articles.

### BACKGROUND OF THE INVENTION

Feminine articles have been used widely for several decades. These articles are generally utilized during menstruation to capture menses and wetness. In general, the user of feminine articles wants to feel clean and dry. Many advancements in feminine article technology have attempted to improve one or both of these aspects. Absorbent core materials have been introduced which can provide enhanced absorbent capacity in a relatively thin article.

However, providing the user of feminine articles the desired experience is a bit more complex and requires more than the above advancements. The user of the feminine article should be provided with an easy to discern application guide which ensures the proper placement /orientation of the feminine article within the panty. If improper placement and/or orientation is utilized, the above advancements of wings and absorbent core materials will likely not provide the intended benefit. Additionally, the article must be securely fastened to the panty. Without the secure fastening of the feminine article to the panty, the feminine article may become detached during use. And similar to improper placement and/or orientation, detachment can negate much of the functionality of the advancements described above. Further, it is desired to make efficient use of the materials in a feminine article, e.g. making sure that the absorbent core is sized and located such that it delivers the most benefits.

US2020375810A1 relates to disposable absorbent articles having a topsheet; a backsheet; and an absorbent system. Further the articles comprise a visual signal visible from a wearer-facing surface. A difference between a length of the disposable absorbent article and a length of the visual signal is less than 35 mm.

US2019314211A1 is concerned with methods of making disposable absorbent articles. The methods include cutting a first and a second absorbent core web in a nesting configuration thereby forming a plurality of discrete first and second absorbent cores. A discrete second absorbent core is joined to a discrete first absorbent core and to the carrier web thereby forming a laminate structure web. A leading edge of the discrete first absorbent core is spaced from a leading edge of the discrete second absorbent core in the machine direction.

Based on the foregoing, it would be beneficial to provide a article which provided easily discernable placement / orientation guides, while using the materials forming the article most efficiently. Thus, resulting in a article that allows the consumer to easily place the most absorbent portions of the article where the most absorbency is needed. Additionally, it would be beneficial to have an absorbent core shaped and sized such that it can efficiently be cut resulting in minimal scrap material. Further, it would be beneficial to have the core shaped such that when the article is folded there is minimal overlap of the core, resulting in a thinly folded product using up less space in a package.

### SUMMARY OF THE INVENTION

The invention provides a method of folding a feminine hygiene article as defined in the claims.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings described below.
Figure 1 is a representation of a feminine article in accordance with the present disclosure.
Figure 2 is a representation of a feminine article in accordance with the present disclosure.
Figure 3 is a representation of a cross section of a feminine article in accordance with the present disclosure, taken along a z-direction plane, of a fluid management layer.
Figure 4 is a representation of a feminine article prior to folding.
Figure 5 is a representation of the nested projection and recess of the absorbent core when the feminine article is in a folded arrangement.
Figure 6 is a representation of the nested projection and recess of the absorbent core when the feminine article is in a folded arrangement.
Figure 7 is a representation of a non-nested absorbent core when the feminine article is in a folded arrangement.

### DETAILED DESCRIPTION OF THE INVENTION

### Definitions

As used herein, the following terms shall have the meaning specified thereafter:
"Absorbent article" refers to wearable devices, which absorb and/or contain liquid, and more specifically, refers to devices, which are placed against or in proximity to the body of the wearer to intercept, absorb and/or contain exudates discharged from the body. Absorbent articles can include diapers, training pants, adult incontinence products (e.g., liners, articles, briefs and underpants) and/or feminine hygiene products, including feminine hygiene articles (also known as, for example, "sanitary napkins", "menstrual articles", "panty liners", etc.).

The term "integrated" as used herein is used to describe fibers of a nonwoven material which have been intertwined, entangled, and/or pushed / pulled in a positive and/or negative z-direction (direction of the thickness of the nonwoven material). Some exemplary processes for integrating fibers of a nonwoven web include spunlacing and needlepunching. Spunlacing (also known as "hydroentangling" or ("hydroenhancing") uses a plurality of high pressure water jets directed at a precursor batt or accumulation of fibers being conveyed along a machine direction, to entangle the fibers. Needlepunching (also known as "needling") involves the use of specially-featured needles to mechanically push and/or pull fibers, of a precursor batt or accumulation of fibers, in a z-direction, to entangle them with other fibers in the batt or accumulation.

The term "carded" as used herein is used to describe structural features of the fluid management layers described herein. A carded nonwoven web is formed of fibers which are cut to a specific finite length, otherwise known as "staple length fibers." Staple length fibers may be of any selected length. For example, staple length fibers may be cut to a length of up to 120 mm, to a length as short as 10 mm. However, if fibers of a particular group are staple length fibers, then the length of each of the fibers in the carded nonwoven is approximately the same, *i.e.* the staple length. Where fibers of more than one composition are included in a nonwoven web, for example, a web including polypropylene fibers and viscose fibers, the length of each fiber of the same composition may be substantially the same, while the respective staple fiber lengths of the respective fiber compositions may differ.

In contrast to staple fibers, filaments such as those produced by spinning, e.g., in a spunbond or meltblown nonwoven web manufacturing processes, are not ordinarily staple length fibers. Instead, these filaments are sometimes characterized as "continuous" fibers, meaning that they are of a relatively long and indeterminate length, not cut to a specific length following spinning, as their staple fiber counterparts are.

"Lateral" - with respect to an absorbent article such as a feminine hygiene article, or a component thereof, refers to a direction parallel to a horizontal line tangent to the front surfaces of the upper portions of wearer's legs proximate the torso, when the article is being worn normally and the wearer has assumed an even, square, normal standing position. A "width" dimension of any component or feature of an article such as a feminine hygiene article is measured along the lateral direction. When the article or component thereof is laid out flat on a horizontal surface, the "lateral" direction corresponds with the lateral direction relative the structure when it is worn, as defined above. With respect to an article such as a feminine hygiene article that is opened and laid out flat on a horizontal planar surface, "lateral" refers to a direction perpendicular to the longitudinal direction and parallel to the horizontal planar surface. With respect to a feminine hygiene article, the term "x-direction" is interchangeable with the term "lateral direction."

The "lateral axis" of an absorbent article such as a feminine hygiene article or component thereof is a lateral line lying in an x-y plane and equally dividing the length of the article or the component when it is laid out flat on a horizontal surface. A lateral axis is perpendicular to a longitudinal axis.

"Longitudinal" - with respect to an absorbent article such as a feminine hygiene article, or a component thereof, refers to a direction perpendicular to the lateral direction. A "length" dimension of any component or feature of the article is measured along the longitudinal direction from its forward extent to its rearward extent. When an article such as a feminine hygiene article or component thereof is laid out flat on a horizontal surface, the "longitudinal" direction is perpendicular to the lateral direction relative the article when it is worn, as defined above. With respect to a feminine hygiene article, the term "y-direction" is interchangeable with the term "longitudinal direction."

The "longitudinal axis" of a feminine hygiene article or component thereof is a longitudinal line lying in an x-y plane and equally dividing the width of the article or component, when the article is laid out flat on a horizontal surface. A longitudinal axis is perpendicular to a lateral axis.

Herein, the term "rayon" is used generically to include any fiber spun from regenerated cellulose, including but not limited to viscose, lyocell, etc.

"x-y plane," with reference to an absorbent article, such as a feminine hygiene article, or component thereof, when laid out flat on a horizontal surface, means any horizontal plane occupied by the horizontal surface or any layer of the article or component. With respect to manufacture or processing of a material web, the term "x-y plane" refers to a plane substantially occupied by a major surface of the material web.

With respect to manufacture or processing of a material web, the term "x-direction" is interchangeable with the term "cross direction."

With respect to manufacture or processing of a material web, the term "y-direction" is interchangeable with the term "machine direction."

"z-direction," with respect to an absorbent article, such as a feminine hygiene article or component thereof, when laid out flat on a horizontal surface, is a direction perpendicular/orthogonal to the x-y plane. With respect to manufacture or processing of a material web, the term "z-direction" refers to a direction orthogonal to an x-y plane substantially occupied by a major surface of the material web.

The terms "top," "bottom," "upper," "lower," "over," "under," "beneath," "superadjacent," "subjacent," and similar terms relating to relative vertical positioning, when used herein to refer to layers, components or other features of an absorbent article such as a feminine hygiene article, are relative the z-direction and are to be interpreted with respect to the article as it would appear when laid out flat on a horizontal surface, with its wearer-facing surface oriented upward and outward-facing surface oriented downward.

With respect to an absorbent article such as a feminine hygiene article, or a component or structure thereof, "wearer-facing" is a relative locational term referring to a feature of the component or structure that when in use that lies closer to the wearer than another feature of the component or structure. For example, a topsheet has a wearer-facing surface that lies closer to the wearer than the opposite, outward-facing surface of the topsheet.

With respect to an absorbent article such as a feminine hygiene article, or a component or structure thereof, "outward-facing" is a relative locational term referring to a feature of the component or structure that when in use that lies farther from the wearer than another feature of the component or structure. For example, a topsheet has an outward-facing surface that lies farther from the wearer than the opposite, wearer-facing surface of the topsheet.

"Predominant," and forms thereof, when used to characterize a quantity of weight, volume, surface area, etc., of an absorbent article or component thereof, constituted by a composition, material, feature, etc., means that a majority of such weight, volume, surface area, etc., of the absorbent article or component thereof is constituted by the composition, material, feature, etc.

### Feminine Hygiene Article

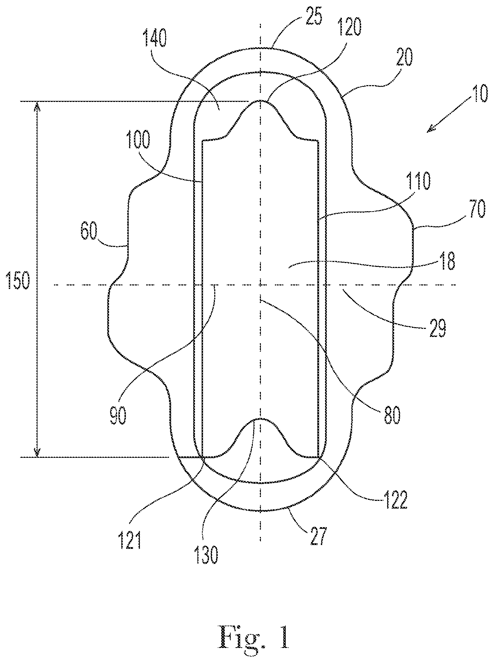

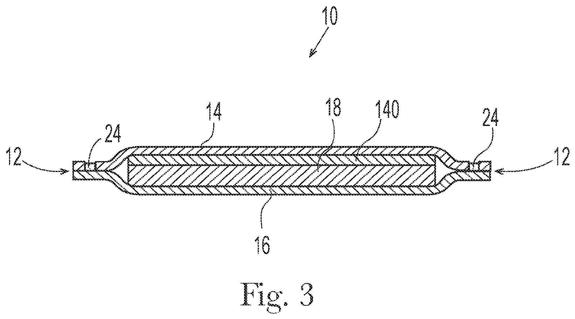

A feminine hygiene article constructed in accordance with the present disclosure is shown in Figure 1. Fig.1 depicts a feminine hygiene article 10. The feminine hygiene article 10 may comprise a chassis 20 and a pair of wings 60 and 70 extending outboard of the chassis 20. The chassis 20 has a first end 25 and a second end 27 and an intermediate region 29 disposed between the first end 25 and the second end 27. The feminine article 10 further comprises a longitudinal centerline 80 and a lateral centerline 90 which is disposed generally perpendicular to the longitudinal centerline 80. The feminine hygiene article 10 may further comprise a topsheet 14, a secondary top sheet 140, a backsheet 16, and an absorbent core 18 disposed between the topsheet and the backsheet (as shown in Fig. 3). The topsheet, backsheet, and absorbent core are discussed in additional detail herein.

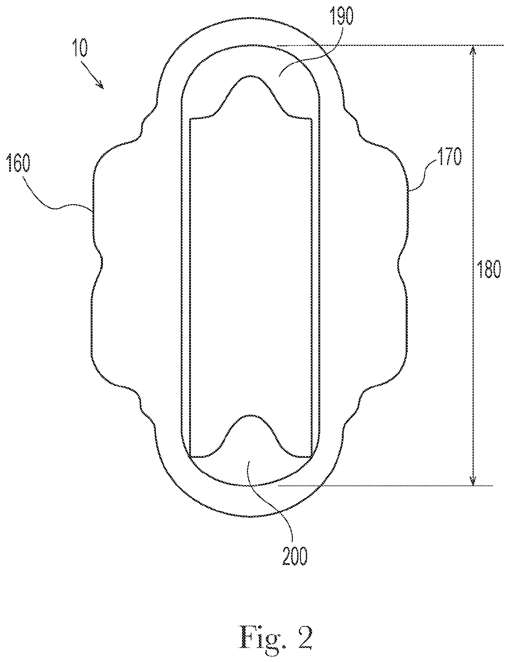

It is desirable to use the materials of a feminine hygiene article most efficiently. One way of achieving an efficient use of materials is to cut them in a manner which reduces excess scrap materials. The absorbent core of the feminine hygiene article contributes to the absorbency of the article, however cutting as described herein both reduces scrap material and does not significantly impact the overall absorbency of the feminine hygiene article. As shown in Figure 1 the absorbent core 18 has a shape that when cut is patterned such that the forwardmost extent of the absorbent core and the rearwardmost extent of the absorbent core are complementary in shape as size. The resulting absorbent core has a pair of substantially parallel and substantially straight longitudinal side edges 100, 110, the side edges terminating at respective forward intersections with a projection 120 which defines a forwardmost extent of the absorbent core, the side edges terminating at respective rearward intersections with a rearwardmost end points 121, 133 and a recess 130, and a total core length measured along the longitudinal direction from the forwardmost point 120 to the rearwardmost point 121 or 122 (whichever is the furthest from the lateral centerline 90) as shown at 150. The rearwardmost extent of the absorbent core includes the rearwardmost end points and the recess. The absorbent core is combined with the secondary topsheet 140. The total absorbent core length is less than the secondary topsheet length. The absorbent core length is from about 75 % to about 99 % of the secondary topsheet length. Alternatively the absorbent core length is from about 75% to about 85% of the secondary topsheet length, alternatively from about 80% to about 95%, and alternatively from about 78% to about 90%. The secondary top sheet length is shown at 180 of Fig. 2 as measured at the forwardmost and rearward most points of the secondary topsheet. The absorbent core can have a total core length of from about 160 mm to about 310 mm, alternatively from about 170 to about 310, alternatively from about 170 to about 250, and alternatively from about 180 mm to about 250 mm. The absorbent core can also have a total core width of from about 55 mm to about 70 mm.

A forward portion of the secondary topsheet length forwardly overhangs 190 the forward projection of the absorbent core; and a rearward portion of the secondary topsheet length rearwardly overhangs 200 the rearward recess of the absorbent core. The forward and rearward overhanging portions 190, 200 of the secondary top sheet provide both additional stability for the feminine hygiene article, extra absorbency, as well as positioning guidance such that when used the product is placed by the user, the target fluid entry zone of the feminine article 10 lines up with the vaginal opening. An intersection between the first imaginary line 80 and the second imaginary line 90 can provide a useful approximation for the target fluid entry zone. The core can be centered on the secondary top sheet so that the impact to the stability of the regions on the forward and rearward most ends of the feminine hygiene article are minimized.

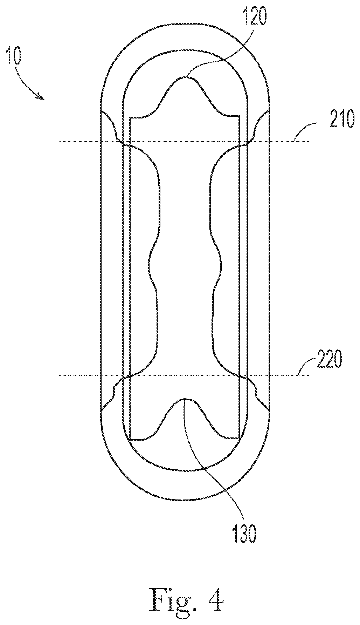

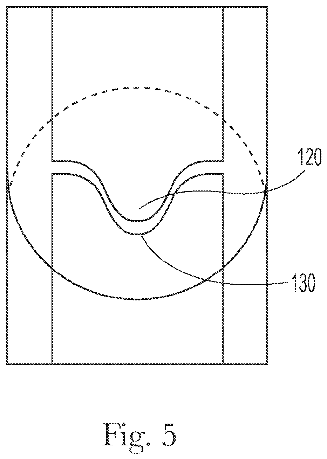

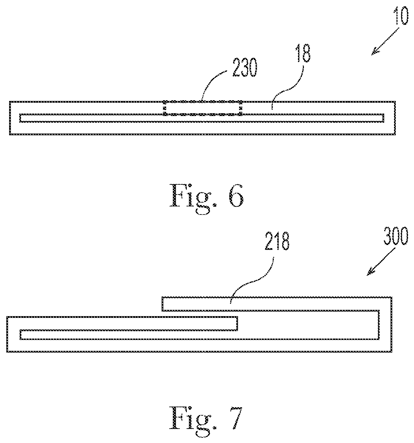

In addition to using less materials this shape/sized absorbent core can result in minimizing packaging materials, as when folded it can result in a thinner product as there is less absorbent core overlap. As shown in Figure 4 when the feminine hygiene article is folded along imaginary lines 210 and 220; the absorbent core projection 120 and recess 130 nest together because they have a complimentary size and shape. This nesting arrangement is shown in Figs. 5 - 6. Figure 6 at 230 demonstrates the feminine hygiene article when folded, such that the absorbent core 18 does not substantially overlap at the forewardmost extent of the absorbent core, which includes the projection, and rearwardmost extent of the absorbent core, which includes the recess, such that the overall folded thickness of the feminine hygiene article is minimized. Whereas, Fig. 7 demonstrates a prior art feminine hygiene article where the forwardmost and rearwardmost terminal ends of the absorbent core 218 overlap, therefore resulting in a thicker folded feminine hygiene article 300.

### Feminine Article Construction

Referring to Figs. 1 - 3, the feminine article 10 is shown. The chassis 20 comprises a liquid permeable topsheet 14, a liquid impermeable, or substantially liquid impermeable, backsheet 16, and an absorbent core 18 positioned intermediate the topsheet and the backsheet. The wings 60 and 70 may be joined to the topsheet, the backsheet, and/or the absorbent core. The wings can be asymmetrical 60, 70 of Fig.1 or symmetrical 160, 170 of Fig. 2.

A feminine hygiene article 10 can have any shape known in the art for feminine hygiene articles, including the generally symmetric "hourglass" shape, as well as pear shapes, bicycle-seat shapes, trapezoidal shapes, wedge shapes or other shapes that have one end wider than the other. Sanitary napkins and pantiliners can also be provided with lateral extensions known in the art as "flaps" or "wings"). Such extensions can serve a number of purposes, including, but not limited to, protecting the wearer's panties from soiling and keeping the sanitary napkin secured in place. The illustrated absorbent article has a body-facing upper side that contacts the user's body during use. The opposite, garment-facing lower side contacts the user's clothing during use.

The upper side of the sanitary napkin 10 generally has a topsheet 14 that can be liquid pervious. The lower side has a backsheet 16 that can generally be liquid impervious and is joined with the topsheet 14 at the edges 12 of the sanitary napkin 10. An absorbent core 18 is positioned between the topsheet 14 and the backsheet 16.

The topsheet 14, the backsheet 16, and the absorbent core 18 can be assembled in a variety of well-known configurations, including so called "tube" products or side flap products. Example sanitary napkin configurations are described generally in U.S. Pat. No. 4,950,264, "Thin, Flexible Sanitary napkin" issued to Osborn on Aug. 21, 1990, U.S. Pat. No. 4,425,130, "Compound Sanitary napkin" issued to DesMarais on Jan. 10, 1984; U.S. Pat. No. 4,321,924, "Bordered Disposable Absorbent Article" issued to Ahr on Mar. 30, 1982; U.S. Pat. No. 4,589,876, and "Shaped Sanitary napkin With Flaps" issued to Van Tilburg on Aug. 18, 1987.

The backsheet 16 and the topsheet 14, as shown in FIG. 3, can be secured together in a variety of ways. Adhesives manufactured by H. B. Fuller Company of St. Paul, Minn. under the designation HL-1258 or H-2031 have been found to be satisfactory. Alternatively, the topsheet 14 and the backsheet 16 can be joined to each other by heat bonding, pressure bonding, ultrasonic bonding, dynamic mechanical bonding, a crimp seal, or by any other suitable securing method. As shown in FIG. 3, a fluid impermeable crimp seal 24 can resist lateral migration ("wicking") of fluid through the edges of the product, inhibiting side soiling of the wearer's undergarments.

As is typical for sanitary napkins and the like, the sanitary napkin 10 of the present disclosure can have panty-fastening adhesive disposed on the garment-facing side of backsheet 16. The panty-fastening adhesive can be any of known adhesives used in the art for this purpose, and can be covered prior to use by a release paper, as is well known in the art. If flaps or wings are present, a panty fastening adhesive can be applied to the garment facing side so as to contact and adhere to the underside of the wearer's panties.

Any suitable absorbent core known in the art may be utilized. The absorbent core may be any absorbent member which is generally compressible, conformable, non-irritating to the wearer's skin, and capable of absorbing and retaining liquids such as urine, menses, and/or other body exudates. The absorbent core may be manufactured from a wide variety of liquid-absorbent materials commonly used in disposable absorbent articles such as comminuted wood pulp which is generally referred to as airfelt. The absorbent core may comprise superabsorbent polymers (SAP) and less than 15%, less than 10%, less than 5%, less than 3%, or less than 1% of airfelt, or be completely free of airfelt. Examples of other suitable absorbent materials comprise creped cellulose wadding, meltblown polymers including coform, chemically stiffened, modified or crosslinked cellulosic fibers, tissue including tissue wraps and tissue laminates, absorbent foams, absorbent sponges, superabsorbent polymers, absorbent gelling materials, or any equivalent material or combinations of materials.

The configuration and construction of the absorbent core may vary (e.g., the absorbent core may have varying caliper zones, a hydrophilic gradient, a superabsorbent gradient, or lower average density and lower average basis weight acquisition zones; or may comprise one or more layers or structures). In some forms, the absorbent core may comprise one or more channels, such as two, three, four, five, or six channels.

The absorbent core of the present disclosure may comprise one or more adhesives, for example, to help immobilize the SAP or other absorbent materials within a core wrap and/or to ensure integrity of the core wrap, in particular when the core wrap is made of two or more substrates. The core wrap may extend to a larger area than required for containing the absorbent material(s) within.

Absorbent cores comprising relatively high amounts of SAP with various core designs are disclosed in U.S. Pat. No. 5,599,335 to Goldman et al., EP 1,447,066 to Busam et al., WO 95/11652 to Tanzer et al., U.S. Pat. Publ. No. 2008/0312622A1 to Hundorf et al., and WO 2012/052172 to Van Malderen.

Other forms and more details regarding channels and pockets that are free of, or substantially free of absorbent materials, such as SAP, within absorbent cores are discussed in greater detail in U.S. Patent Application Publication Nos. 2014/0163500, 2014/0163506, and 2014/0163511, all published on June 12, 2014.

Other suitable materials for use in absorbent cores comprise open celled foams or pieces thereof. The use of foams in absorbent cores is described in additional detail in U.S. Patent Nos. 6,410,820; 6,107,356; 6,204,298; 6,207,724; 6,444,716; 8,211,078, and 8,702,668.

In some forms, the absorbent core structure may comprise a heterogeneous mass layer or may utilize methods or parameters such as those described in US patent application nos. 14/715,984, filed May 19, 2015; US Patent Application No. 14/750,399, June 25, 2015; US Patent Application No. 14/751,969 filed June 26, 2015; US Patent Application No. 15/078,132 filed March 23, 2016; US Patent Application No. 14/750,596 filed June 25, 2015; US Patent Application No. 15/084,902 filed March 30, 2016; US Patent Application No. 15/343,989 filed November 4, 2016; US Patent Application No. 15/344,273 filed November 4, 2016; US Patent Application No. 15/344,294 filed November 4, 2016; US Patent Application No. 14/704,110 filed May 5, 2015; US Patent Application No. 15/194,894 filed June 28, 2016; US Patent Application No. 15/344,050 filed November 4, 2016; US Patent Application No. 15/344,117 filed November 4, 2016; US Patent Application No. 15/344,177 filed November 4, 2016; US Patent Application No. 15/344,198 filed November 4, 2016; US Patent Application No. 15/344,221 filed November 4, 2016; US Patent Application No. 15/344,239 filed November 4, 2016; US Patent Application No. 15/344,255 filed November 4, 2016; US Patent Application No. 15/464,733 filed November 4, 2016; US Provisional Patent Application No. 62/437,208 filed December 21, 2016; US Provisional Patent Application No. 62/437,225 filed December 21, 2016; US Provisional Patent Application No. 62/437,241 filed December 21, 2016; or US Provisional Patent Application No. 62/437,259 filed December 21, 2016. The heterogeneous mass layer has a depth, a width, and a height.

In some forms, a combination of absorbent core materials may be utilized. For example, forms are contemplated where a first layer of an absorbent core comprises a foam material or pieces thereof as described previously and a second layer of an absorbent core comprises an airlaid material. Such combinations are described in U.S. Patent Publication No. 2014/0336606 and U.S. Patent No. 9,649,228.

The feminine article comprises a secondary topsheet and/or an acquisition layer positioned between the topsheet and the absorbent core. One suitable example of a secondary topsheet is with regard to a spunlace nonwoven. Suitable spunlace nonwovens are discussed in additional detail in U.S. Patent Publication No. 2015/0351976. In some forms, the secondary topsheet may comprise superabsorbent similar to the superabsorbent in the absorbent core or different than the absorbent core.

The backsheet can comprise a liquid impervious film. The backsheet can be impervious to liquids (e.g., body fluids) and can be typically manufactured from a thin plastic film. However, typically the backsheet can permit vapours to escape from the disposable article. In an embodiment, a microporous polyethylene film can be used for the backsheet. A suitable microporous polyethylene film is manufactured by Mitsui Toatsu Chemicals, Inc., Nagoya, Japan and marketed in the trade as PG-P.

One suitable material for the backsheet can be a liquid impervious thermoplastic film having a thickness of from about 0.012 mm (0.50 mil) to about 0.051 mm (2.0 mils), for example including polyethylene or polypropylene. Typically, the backsheet can have a basis weight of from about 5 g/m² to about 35 g/m². However, it should be noted that other flexible liquid impervious materials may be used as the backsheet. Herein, "flexible" refers to materials which are compliant and which will readily conform to the general shape and contours of the wearer's body.

The backsheet can be typically positioned adjacent an outer-facing surface of the absorbent core and can be joined thereto by any suitable attachment device known in the art. For example, the backsheet may be secured to the absorbent core by a uniform continuous layer of adhesive, a patterned layer of adhesive, or an array of separate lines, spirals, or spots of adhesive. Illustrative, but non-limiting adhesives, include adhesives manufactured by H. B. Fuller Company of St. Paul, Minn., U.S.A., and marketed as HL-1358J. An example of a suitable attachment device including an open pattern network of filaments of adhesive is disclosed in U.S. Pat. No. 4,573,986 entitled "Disposable Waste-Containment Garment", which issued to Minetola et al. on Mar. 4, 1986. Another suitable attachment device including several lines of adhesive filaments swirled into a spiral pattern is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment device may include heat bonds, thermal fusion bonds, pressure bonds, ultrasonic bonds, dynamic mechanical bonds, or any other suitable attachment device or combinations of these attachment devices. The backsheet may be additionally secured to the topsheet by any of the above-cited attachment devices / methods.

The topsheet is positioned adjacent a body-facing surface of the feminine article. The topsheet may be joined to the backsheet by attachment methods (not shown) such as those well known in the art. The topsheet and the backsheet may be joined directly to each other in the feminine article periphery and may be indirectly joined together by directly joining them to the absorbent core by any suitable attachment method.

The topsheet may be compliant, soft feeling, and non-irritating to the wearer's skin. Further, the topsheet may be liquid pervious permitting liquids (e.g., urine, menses) to readily penetrate through its thickness. Some suitable examples of topsheet materials include films, nonwovens, laminate structures including film / nonwoven layers, film / film layers, and nonwoven / nonwoven layers. Other exemplary topsheet materials and designs are disclosed in U.S. Patent Application Publication Nos. 2016/0129661, 2016/0167334, and 2016/0278986.

As noted previously, the feminine article may comprise a secondary topsheet or an acquisition layer disposed between the topsheet and the absorbent core. The secondary topsheet may comprise a first end and an opposing second end and a pair of longitudinally opposing side edges and connecting the first end and the second end. As shown, the secondary topsheet may be asymmetrical or symmetrical about the longitudinal centerline 80.

The wings of the feminine hygiene articles of the present disclosure may be integrally formed as part of the topsheet. In some forms, the wings may be integrally formed as part of the backsheet. In some forms, the wings may be integrally formed as part of the topsheet and the backsheet. In some forms, the wings may be integrally formed with additional layers - described herein - of the feminine hygiene article. Yet in other forms, the wings may be formed discretely and joined to the chassis.

### Test and Measurement Methods

### Caliper

The caliper, or thickness, of a test specimen is measured as the distance between a reference platform on which the specimen rests and a pressure foot that exerts a specified amount of pressure onto the specimen over a specified amount of time. All measurements are performed in a laboratory maintained at 23 °C ± 2 C° and 50% ± 2% relative humidity and test specimens are conditioned in this environment for at least 2 hours prior to testing.

Caliper is measured with a manually-operated micrometer equipped with a pressure foot capable of exerting a steady pressure of 0.50 kPa ± 0.01 kPa onto the test specimen. The manually-operated micrometer is a dead-weight type instrument with readings accurate to 0.01 mm. A suitable instrument is Mitutoyo Series 543 ID-C Digimatic, available from VWR International, or equivalent. The pressure foot is a flat ground circular movable face with a diameter that is smaller than the test specimen and capable of exerting the required pressure. A suitable pressure foot has a diameter of 25.4 mm, however a smaller or larger foot can be used depending on the size of the specimen being measured. The test specimen is supported by a horizontal flat reference platform that is larger than and parallel to the surface of the pressure foot. The system is calibrated and operated per the manufacturer's instructions.

Obtain a test specimen by removing it from an absorbent article, if necessary. When excising the test specimen from an absorbent article, use care to not impart any contamination or distortion to the test specimen layer during the process. The test specimen is obtained from an area free of folds or wrinkles, and it must be larger than the pressure foot.

To measure caliper, first zero the micrometer against the horizontal flat reference platform. Place the test specimen on the platform with the test location centered below the pressure foot. Gently lower the pressure foot with a descent rate of 3.0 mm ± 1.0 mm per second until the full pressure is exerted onto the test specimen. Wait 5 seconds and then record the caliper of the test specimen to the nearest 0.001 mm. In like fashion, repeat for a total of ten replicate test specimens. Calculate the arithmetic mean for all caliper measurements and report as Caliper to the nearest 0.001 mm.

### Caliper factor

The caliper factor, as mentioned previously is the caliper per 10 gsm of basis weight of the sample. So, the equation is caliper / (basis weight/10).

### Basis Weight

The basis weight of a sample of sheet or web material is the mass (in grams) per unit area (in square meters) of a single layer of the material. If it is not otherwise known or available, basis weight may be measured using EDANA compendial method NWSP 130.1. The mass of the test sample is cut to a known area, and the mass of the sample is determined using an analytical balance accurate to 0.0001 grams. All measurements are performed in a laboratory maintained at 23 °C ± 2 C° and 50% ± 2% relative humidity and test samples are conditioned in this environment for at least 2 hours prior to testing.

Measurements are made on test samples taken from rolls or sheets of the raw material, or test samples obtained from a material layer removed from an absorbent article. When excising the material layer from an absorbent article, use care to not impart any contamination or distortion to the layer during the process. The excised layer should be free from residual adhesive. To ensure that all adhesive is removed, soak the layer in a suitable solvent that will dissolve the adhesive without adversely affecting the material itself. One such solvent is THF (tetrahydrofuran, CAS 109-99-9, for general use, available from any convenient source). After the solvent soak, the material layer is allowed to thoroughly air dry in such a way that prevents undue stretching or other deformation of the material. After the material has dried, a test specimen is obtained. The test specimen must be as large as possible so that any inherent material variability is accounted for.

Measure the dimensions of the single layer test specimen using a calibrated steel metal ruler traceable to NIST, or equivalent. Calculate the Area of the test specimen and record to the nearest 0.0001 square meter. Use an analytical balance to obtain the Mass of the test specimen and record to the nearest 0.0001 gram. Calculate Basis Weight by dividing Mass (in grams) by Area (in square meters) and record to the nearest 0.01 grams per square meter (gsm). In like fashion, repeat for a total of ten replicate test specimens. Calculate the arithmetic mean for Basis Weight and report to the nearest 0.01 grams/square meter.

## Claims

1. A method of folding a feminine hygiene article (10), the feminine hygiene article (10) comprising:
a liquid permeable topsheet (14);
a secondary topsheet (140) beneath the topsheet (14), having a secondary topsheet length measured along a longitudinal direction;
a liquid impermeable backsheet (16);
an absorbent core (18) disposed between the topsheet (14) and the backsheet (16); and said absorbent core (18) disposed between the secondary topsheet (140) and the backsheet (16), the absorbent core (18) having a pair of substantially parallel and substantially straight longitudinal side edges (100, 110), the side edges terminating at respective forward intersections with a projection (120) of the absorbent core (18), the side edges (100, 110) terminating at respective rearward intersections with a rearward recess (130) of the absorbent core (18), and a total core length measured along the longitudinal direction at the forwardmost and rearward most points of the absorbent core, wherein:
the total core length is less than the secondary topsheet length;
a forward portion of the secondary topsheet length forwardly overhangs the forward projection (120);
a rearward portion of the secondary topsheet length rearwardly overhangs the rearward recess (130);
the total core length as measured at the forwardmost and rearward most points of the absorbent core (18) is from 75 % to 99 % of the secondary topsheet length, preferably from 75% to 85%, as measured at the forwardmost and rearward most points of the secondary topsheet (140); and
the forward projection (120) and the rearward recess (130) are substantially complementary in size and shape;
wherein the article (10) is folded such that the forward projection (120) nests in the rearward recess (130).

2. The method of claim 1, wherein the height of projection and depth of the recess are each from 15 mm to 25mm.

3. The method of any one of the preceding claims, wherein the height of the projection and depth of the recess are each from 15 mm to 20 mm.

4. The method of any one of the preceding claims, wherein the projection (120) has a convex shape.

5. The method of any one of the preceding claims, wherein the recess (130) has a concave shape.

6. The method of any one of the preceding claims, wherein the total core length is from 160 mm to 310 mm, preferably from 170 mm to 310 mm, more preferably from 170 mm to 250 mm, most preferably from 180 mm to 250 mm.

7. The method of any one of the preceding claims, wherein the total core width is from 55 mm to 70 mm.

8. The method of any one of the preceding claims, wherein the secondary top sheet (140) has a straight cut edge along the longitudinal side edges (100, 110) of the absorbent core (18).

9. The method of any one of the preceding claims, wherein the secondary top sheet (140) has an hourglass shaped edge along the longitudinal side edges (100, 110) of the absorbent core (18).

## Patentansprüche

1. Verfahren zum Falten eines Damenhygieneartikels (10), der Damenhygieneartikel (10) umfassend:
eine flüssigkeitsdurchlässige Oberschicht (14);
eine sekundäre Oberschicht (140) unter der Oberschicht (14), die eine entlang einer Längsrichtung gemessene Länge der sekundären Oberschicht aufweist;
eine flüssigkeitsundurchlässige Unterschicht (16);
einen Absorptionskern (18), der zwischen der Oberschicht (14) und der Unterschicht (16) angeordnet ist; und
wobei der Absorptionskern (18) zwischen der sekundären Oberschicht (140) und der Unterschicht (16) angeordnet ist, der Absorptionskern (18) ein Paar im Wesentlichen paralleler und im Wesentlichen gerader Längsseitenränder (100, 110) aufweist, die Seitenränder an jeweiligen vorderen Schnittpunkten mit einem Vorsprung (120) des Absorptionskerns (18) enden, die Seitenränder (100, 110) an jeweiligen hinteren Schnittpunkten mit einer hinteren Aussparung (130) des Absorptionskerns (18) enden und eine Gesamtkernlänge entlang der Längsrichtung an den vordersten und hintersten Punkten des Absorptionskerns gemessen wird, wobei:
die Gesamtkernlänge geringer als die Länge der sekundären Oberschicht ist;
ein vorderer Abschnitt der Länge der sekundären Oberschicht den vorderen Vorsprung (120) nach vorne überragt;
ein hinterer Abschnitt der Länge der zweiten Oberschicht die hintere Aussparung (130) nach hinten überragt;
die Gesamtkernlänge, gemessen an den vordersten und hintersten Punkten des Absorptionskerns (18), von 75 % bis 99 % der Länge der sekundären Deckschicht, vorzugsweise von 75 % bis 85 %, gemessen an den vordersten und hintersten Punkten der sekundären Deckschicht (140), beträgt; und
der vordere Vorsprung (120) und die hintere Aussparung (130) in Größe und Form im Wesentlichen komplementär sind;
wobei der Artikel (10) derart gefaltet ist, dass der vordere Vorsprung (120) in der hinteren Aussparung (130) liegt.

2. Verfahren nach Anspruch 1, wobei die Höhe des Vorsprungs und die Tiefe der Aussparung jeweils von 15 mm bis 25 mm betragen.

3. Verfahren nach einem der vorstehenden Ansprüche, wobei die Höhe des Vorsprungs und die Tiefe der Aussparung jeweils von 15 mm bis 20 mm betragen.

4. Verfahren nach einem der vorstehenden Ansprüche, wobei der Vorsprung (120) eine konvexe Form aufweist.

5. Verfahren nach einem der vorstehenden Ansprüche, wobei die Aussparung (130) eine konkave Form aufweist.

6. Verfahren nach einem der vorstehenden Ansprüche, wobei die Gesamtkernlänge von 160 mm bis 310 mm, vorzugsweise von 170 mm bis 310 mm, mehr bevorzugt von 170 mm bis 250 mm, am meisten bevorzugt von 180 mm bis 250 mm beträgt.

7. Verfahren nach einem der vorstehenden Ansprüche, wobei die Gesamtkernbreite von 55 mm bis 70 mm beträgt.

8. Verfahren nach einem der vorstehenden Ansprüche, wobei die sekundäre Oberschicht (140) eine gerade Schnittkante entlang der Längsseitenränder (100, 110) des Absorptionskerns (18) aufweist.

9. Verfahren nach einem der vorstehenden Ansprüche, wobei die sekundäre Oberschicht (140) entlang der Längsseitenränder (100, 110) des Absorptionskerns (18) einen sanduhrförmigen Rand aufweist.

## Revendications

1. Procédé de pliage d'un article d'hygiène féminine (10), l'article d'hygiène féminine (10) comprenant :
une feuille de dessus perméable aux liquides (14) ;
une feuille de dessus secondaire (140) en dessous de la feuille de dessus (14), ayant une longueur de feuille de dessus secondaire mesurée le long d'une direction longitudinale ;
une feuille de fond imperméable aux liquides (16) ;
une âme absorbante (18) disposée entre la feuille de dessus (14) et la feuille de fond (16) ; et
ladite âme absorbante (18) étant disposée entre la feuille de dessus secondaire (140) et la feuille de fond (16), l'âme absorbante (18) ayant une paire de bords latéraux longitudinaux (100, 110) sensiblement parallèles et sensiblement droits, les bords latéraux se terminant à des intersections avant respectives par une saillie (120) de l'âme absorbante (18), les bords latéraux (100, 110) se terminant à des intersections arrière respectives par un évidement arrière (130) de l'âme absorbante (18), et une longueur totale d'âme mesurée le long de la direction longitudinale au niveau de points le plus à l'avant et le plus à l'arrière de l'âme absorbante, dans lequel :
la longueur totale d'âme est inférieure à la longueur de feuille de dessus secondaire ;
une partie avant de la longueur de feuille de dessus secondaire surplombe vers l'avant la saillie avant (120) ;
une partie arrière de la longueur de feuille de dessus secondaire surplombe vers l'arrière l'évidement arrière (130) ;
la longueur totale d'âme telle que mesurée au niveau des points le plus à l'avant et le plus à l'arrière de l'âme absorbante (18) va de 75 % à 99 % de la longueur de feuille de dessus secondaire, de préférence de 75 % à 85 %, telle que mesurée au niveau des points le plus à l'avant et le plus à l'arrière de la feuille de dessus secondaire (140) ; et
la saillie avant (120) et l'évidement arrière (130) sont sensiblement complémentaires en taille et en forme ;
dans lequel l'article (10) est plié de telle sorte que la saillie avant (120) s'imbrique dans l'évidement arrière (130).

2. Procédé selon la revendication 1, dans lequel la hauteur de saillie et la profondeur de l'évidement vont chacune de 15 mm à 25 mm.

3. Procédé selon l'une quelconque des revendications précédentes, dans lequel la hauteur de la saillie et la profondeur de l'évidement vont chacune de 15 mm à 20 mm.

4. Procédé selon l'une quelconque des revendications précédentes, dans lequel la saillie (120) a une forme convexe.

5. Procédé selon l'une quelconque des revendications précédentes, dans lequel l'évidement (130) a une forme concave.

6. Procédé selon l'une quelconque des revendications précédentes, dans lequel la longueur totale d'âme va de 160 mm à 310 mm, de préférence de 170 mm à 310 mm, plus préférablement de 170 mm à 250 mm, le plus préférablement de 180 mm à 250 mm.

7. Procédé selon l'une quelconque des revendications précédentes, dans lequel la largeur totale d'âme va de 55 mm à 70 mm.

8. Procédé selon l'une quelconque des revendications précédentes, dans lequel la feuille de dessus secondaire (140) a un bord à découpe droite le long des bords latéraux longitudinaux (100, 110) de l'âme absorbante (18).

9. Procédé selon l'une quelconque des revendications précédentes, dans lequel la feuille de dessus secondaire (140) a un bord en forme en sablier le long des bords latéraux longitudinaux (100, 110) de l'âme absorbante (18).
